# EUROPEAN PATENT APPLICATION

(11) **EP 1 406 197 A2**
(43) Date of publication of application: **07.04.2004**
(21) Application number: 03017441.1
(22) Date of filing: 01.08.2003
(51) Int. Cl.: G06F 19/00

(54) **Apparatus, system and program for controlling the operation of photographing a medical image**

(30) Priority: 16.08.2002 JP 2002237690
(71) Applicant: KONICA CORPORATION, Tokyo 163-0512 (JP)
(72) Inventor: Motoki, Wataru, Hachioji-shi, Tokyo 192-8505 (JP)
(74) Representative: Gille Hrabal Struck Neidlein Prop Roos

(57) **Abstract**

A medical image control apparatus for controlling a cassette-type medical image photographing apparatus and at least one of an upright-type medical image photographing apparatus and a bucky-table-type medical image photographing apparatus. The medical image control apparatus has: a display control section for displaying a photographing management screen on a display section, the photographing management screen capable of managing medical image photographing operation, and a photographing operation control section for controlling medical image photographing operation of a medical image photographing apparatus ordered on the photographing management screen, wherein the display control section individually displays medical images within photographing operation management areas corresponding to the cassette-type medical image photographing apparatus and the at least one of an upright-type medical image photographing apparatus and a bucky-table-type medical image photographing apparatus, and the photographing operation control section controls medical image photographing operation according to orders on the photographing operation management areas.

## Description

### BACKGROUND OF THE INVENTION

### Field of the Invention

The invention relates to an apparatus, a system and a program for controlling operation of photographing a medical image in a medical image photographing apparatus.

### Description of Related Art

In a field of medicine, a medical image photographing system for photographing a medical image by using a medical image photographing apparatus such as CT (Computed Tomography), CR (Computed Radiography) or the like and obtaining the photographed medical image as a digital image has been being developed.

Concretely, in the medical image photographing apparatus, a medical image conversion panel wherein a photostimulable phosphor layer is formed on a support member is in use. First, the photostimulable phosphor sheet in the medical image conversion panel absorbs radiation passed through a subject for accumulating radiation energy corresponding to amount of the radiation passed through each part of the subject, and then a latent image is formed in the photostimulable phosphor sheet. After that, the photostimulable phosphor layer is scanned by excitation light such as an infrared ray or the like for emitting stimulated light. Then, the stimulated light is photoelectrically converted for obtaining a medical image signal. The medical image signal obtained as mentioned, after being applied an image process on, is outputted onto film or to an output apparatus such as a CRT or the like to be visualized, or stored in a filing apparatus such as a server or the like along with patient information for medical service purposes.

In the above-mentioned medical image photographing system, a controller controls a plurality of medical image photographing apparatuses (hereafter, it is written as "reader"). Based on reservation information sent from an information system such as an HIS (Hospital Information System), an RIS (Radiology Information System) or the like managing information in a department of radiology or a hospital, a medical image is photographed. Here, the medical image photographing system comprises a plurality of readers according to photographing usage, and each reader is controlled according to its control pattern by a plurality of medical image control apparatuses (hereafter, it is written as "controller").

For example, in a reader for photographing for an upright position and a reader for photographing for a bucky table position, since photographing is performed with a photostimulable phosphor plate incorporated in the apparatus, it is necessary to execute a process for erasing image data that has been generated from the last time photographing before performing photographing newly. Therefore, detailed control by the controller is necessary. However, the readers for photographing for an upright position and a bucky table position can be controlled by the same controller because they have the same control pattern. On the other hand, since a reader of cassette-type using a cassette incorporating the photostimulable phosphor sheet therein is able to photograph continuously with a plurality of cassettes set simultaneously, detailed control by the controller is not necessary before performing photographing newly. Therefore, the reader of cassette-type is controlled by a controller other than the one controlling the readers of upright-type and bucky-table - type.

By the way, in the above-mentioned medical image photographing system, since photographing is performed to one patient with a plurality of readers, a photographic technician needs to perform photographing while the plurality of readers are controlled by a plurality of controllers. Therefore, control operation to each reader is very complicated. For example, when patient information of a patient to be photographed is incorrect, it is necessary to correct the patient information to each controller. As a result, it takes lots of labor and time on the operation inefficiently. Further, installation of the plurality of controllers requires more cost for maintaining the readers and more space in a limited area of a hospital.

### SUMMARY OF THE INVENTION

An object of the present invention is to provide a medical image control apparatus, a medical image control system and a program capable of controlling a plurality of readers comprised in a medical image control system efficiently and executing a photographing process promptly.

In accordance with a first aspect of the present invention, a medical image control apparatus for controlling a cassette-type medical image photographing apparatus and at least one of an upright-type medical image photographing apparatus and a bucky-table-type medical image photographing apparatus, the medical image control apparatus comprises: a display control section for displaying a photographing management screen on a display section, the photographing management screen capable of managing medical image photographing operation of each of the cassette-type medical image photographing apparatus and the at least one of an upright-type medical image photographing apparatus and a bucky-table-type medical image photographing apparatus based on photographing reservation information therewithin, and a photographing operation control section for controlling medical image photographing operation of a medical image photographing apparatus ordered on the photographing management screen, wherein the display control section individually displays medical images within photographing operation management areas corresponding to the cassette-type medical image photographing apparatus and the at least one of an upright-type medical image photographing apparatus and a bucky-table-type medical image photographing apparatus, the medical images read by the cassette-type medical image photographing apparatus and the at least one of an upright-type medical image photographing apparatus and a bucky-table-type medical image photographing apparatus corresponding, and the photographing operation control section controls medical image photographing operation corresponding to each of the cassette-type medical image photographing apparatus and the at least one of an upright-type medical image photographing apparatus and a bucky-table-type medical image photographing apparatus, according to an order on each of the photographing operation management areas within the photographing management screen.

In accordance with a second aspect of the present invention, in medical image control system comprising a cassette-type medical image photographing apparatus and at least one of an upright-type medical image photographing apparatus and a bucky-table-type medical image photographing apparatus and a medical image control apparatus for controlling the cassette-type medical image photographing apparatus and the at least one of an upright-type medical image photographing apparatus and a bucky-table-type medical image photographing apparatus, the medical image control apparatus comprises: a display control section for displaying a photographing management screen on a display section, the photographing management screen capable of managing medical image photographing operation of each of the cassette-type medical image photographing apparatus and the at least one of an upright-type medical image photographing apparatus and a bucky-table-type medical image photographing apparatus based on photographing reservation information therewithin, and a photographing operation control section for controlling medical image photographing operation of a medical image photographing apparatus ordered on the photographing management screen, wherein the display control section individually displays medical images within photographing operation management areas corresponding to the cassette-type medical image photographing apparatus and the at least one of an upright-type medical image photographing apparatus and a bucky-table-type medical image photographing apparatus, the medical images read by the cassette-type medical image photographing apparatus and the at least one of an upright-type medical image photographing apparatus and a bucky-table-type medical image photographing apparatus corresponding, and the photographing operation control section controls medical image photographing operation corresponding to each of the cassette-type medical image photographing apparatus and the at least one of an upright-type medical image photographing apparatuses and a bucky-table-type medical image photographing apparatus, according to an order on each of the photographing operation management areas within the photographing management screen.

In accordance with a third aspect of the present invention, a program, when the program is loaded onto a medical image control apparatus for controlling a cassette-type medical image photographing apparatus and at least one of an upright-type medical image photographing apparatus and a bucky-table-type medical image photographing apparatus, the program makes the medical image control apparatus execute: displaying a photographing management screen on a display section, the photographing management screen capable of managing medical image photographing operation of each of the cassette-type medical image photographing apparatus and the at least one of an upright-type medical image photographing apparatus and a bucky-table-type medical image photographing apparatus based on photographing reservation information therewithin, and controlling medical image photographing operation of a medical image photographing apparatus ordered on the photographing management screen, wherein the displaying a photographing management screen on a display section includes individually displaying medical images within photographing operation management areas corresponding to the cassette-type medical image photographing apparatus and the at least one of an upright-type medical image photographing apparatus and a bucky-table-type medical image photographing apparatus, the medical images read by the cassette-type medical image photographing apparatus and the at least one of an upright-type medical image photographing apparatus and a bucky-table-type medical image photographing apparatus corresponding, and the controlling medical image photographing operation of a medical image photographing apparatus includes controlling medical image photographing operation corresponding to each of the cassette-type medical image photographing apparatus and the at least one of an upright-type medical image photographing apparatuses and a bucky-table-type medical image photographing apparatus, according to an order on each of the photographing operation management areas within the photographing management screen.

According to the apparatus of the first aspect, the system of the second aspect or the program of the third aspect of the present invention, since it is possible to manage a cassette-type medical image photographing apparatus and at least one of an upright-type medical image photographing apparatuses and a bucky-table-type medical image photographing apparatus on a photographing management screen capable of managing the cassette-type medical image photographing apparatus and the at least one of an upright-type medical image photographing apparatuses and a bucky-table-type medical image photographing apparatus within a same screen, it is possible to provide an user-friendly medical image control apparatus. Further, since one medical image control apparatus can manage the cassette-type medical image photographing apparatus and the at least one of an upright-type medical image photographing apparatuses and a bucky-table-type medical image photographing apparatus, it is not necessary to provide a controller for each of the cassette-type medical image photographing apparatus and the at least one of an upright-type medical image photographing apparatuses and a bucky-table-type medical image photographing apparatus, and therefore it is possible to reduce maintenance cost of the medical image control apparatus.

Further, in addition to above-mentioned advantages of the present invention, since one medical image control apparatus can control apparatuses having different control patterns such as medical image photographing apparatuses of upright-type, bucky-table-type and cassette-type controlled by the medical image control apparatus, it is not necessary to operate a plurality of medical image control apparatuses respectively for controlling a plurality of medical image photographing apparatuses. Therefore, it is possible to improve operation efficiency drastically. Further, since photographing operation management areas corresponding to each medical image photographing apparatus are individually displayed within the photographing management screen, it is possible to provide a display screen having good visibility.

Preferably, in the apparatus of the first aspect of the present invention, the order on each of the photographing operation management areas includes at least one of an order of exiting operation normally and an order of aborting operation.

Preferably, in the system of the second aspect of the present invention, the order on each of the photographing operation management areas includes at least one of an order of exiting operation normally and an order of aborting operation.

Preferably, in the program of the third aspect of the present invention, the order on each of the photographing operation management areas includes at least one of an order of exiting operation normally and an order of aborting operation.

Preferably, the apparatus of the first aspect of the present invention further comprises a communication control section for receiving the photographing reservation information from an information system.

More preferably, the photographing operation control section judges whether the cassette-type medical image photographing apparatus capable of performing reading operation when the cassette-type medical image photographing apparatus is selected by operation, and the photographing operation control section controls registration of a code of a cassette set in the cassette-type medical image photographing apparatus and the photographing reservation information received, the code and the photographing reservation information are related, when judging the cassette-type medical image photographing apparatus is capable of performing the reading operation.

Preferably, in the system of the second aspect of the present invention, the medical image control apparatus further comprises a communication control section for receiving the photographing reservation information from an information system.

More preferably, the photographing operation control section judges whether the cassette-type medical image photographing apparatus is capable of performing reading operation when the cassette-type medical image photographing apparatus is selected by operation, and the photographing operation control section controls registration of a code of a cassette set in the cassette-type medical image photographing apparatus and the photographing reservation information received, the code and the photographing reservation information are related, when judging the cassette-type medical image photographing apparatus is capable of performing the reading operation.

Preferably, the program of the third aspect of the present invention further makes the medical image control apparatus execute receiving the photographing reservation information from an information system.

More preferably, the controlling medical image photographing operation of a medical image photographing apparatus includes judging whether the cassette-type medical image photographing apparatus is capable of performing reading operation when the cassette-type medical image photographing apparatus is selected by operation, and the controlling medical image photographing operation of a medical image photographing apparatus includes controlling registration of a code of a cassette set in the cassette-type medical image photographing apparatus and the photographing reservation information received, the code and the photographing reservation information are related, when judging the cassette-type medical image photographing apparatus is capable of performing the reading operation.

Preferably, the apparatus of the first aspect of the present invention further comprises a display section for displaying any one of the medical images displayed on the photographing management screen by operation.

Preferably, in the system of the second aspect of the present invention, the medical image control apparatus further comprises a display section for displaying any one of the medical images displayed on the photographing management screen by operation.

Preferably, the program of the third aspect of the present invention further makes the medical image control apparatus execute displaying any one of the medical images displayed on the photographing management screen by operation.

Preferably, in the apparatus of the first aspect of the present invention, the photographing operation control section controls reading operation of the cassette-type medical image photographing apparatus and the at least one of an upright-type medical image photographing apparatuses and a bucky-table-type medical image photographing apparatus according to the photographing reservation information.

Preferably, in the system of the second aspect of the present invention, the photographing operation control section controls reading operation of the cassette-type medical image photographing apparatus and the at least one of an upright-type medical image photographing apparatuses and a bucky-table-type medical image photographing apparatus according to the photographing reservation information.

Preferably, in the program of the third aspect of the present invention, the controlling medical image photographing operation of a medical image photographing apparatus includes controlling reading operation of the cassette-type medical image photographing apparatus and the at least one of an upright-type medical image photographing apparatuses and a bucky-table-type medical image photographing apparatus according to the photographing reservation information.

### BRIEF DESCRIPTION OF THE DRAWINGS

The present invention will become more fully understood from the detailed description given hereinafter and the accompanying drawing given by way of illustration only, and thus are not intended as a definition of the limits of the present invention, and wherein:
FIG. 1 is a conceptual diagram showing a system structure of a medical image control system 100 in an embodiment to which the present invention is applied,
FIG. 2 is a block diagram showing a substantial structure of a controller 1 shown in FIG. 1,
FIG. 3 is a block diagram showing a substantial structure of a reader 2 shown in FIG. 1,
FIGs. 4A and 4B are a flow chart showing a photographing management process executed by a control unit 11 shown in FIG. 2, and
FIG. 5 is a view showing a photographing management screen 121 displayed on a display unit 13 in Step S2 shown in FIG. 4A.

### AN EMBODIMENT OF THE INVENTION

Hereinafter, an embodiment of the present invention will be explained in detail with reference to figures. However, a range of the present invention is not limited to examples shown in FIGs.

Here, the relationship between a medical image control system in regard to the present invention and a medical image control system 100 of the present embodiment will be specified. In other words, the medical image control apparatus of the present invention corresponds to a controller 1 of the present embodiment, and a medical image photographing apparatus of the present invention corresponds to readers 2, 3 and 4 of the present embodiment.

Further, the relationship between components of the medical image control apparatus of the present invention and components of the controller 1 of the present embodiment will be specified. A display section of the present invention corresponds to a display unit 13 of the present embodiment, and a display control section and a photographing operation control section of the present invention correspond to a control unit 11 of the present embodiment. Further, as one example of photographing reservation information of the present invention, study information of the present embodiment corresponds, and as one example of a photographing management screen, a photographing management screen 121 of the present embodiment corresponds.

Hereinafter, a structure of the present embodiment will be explained.

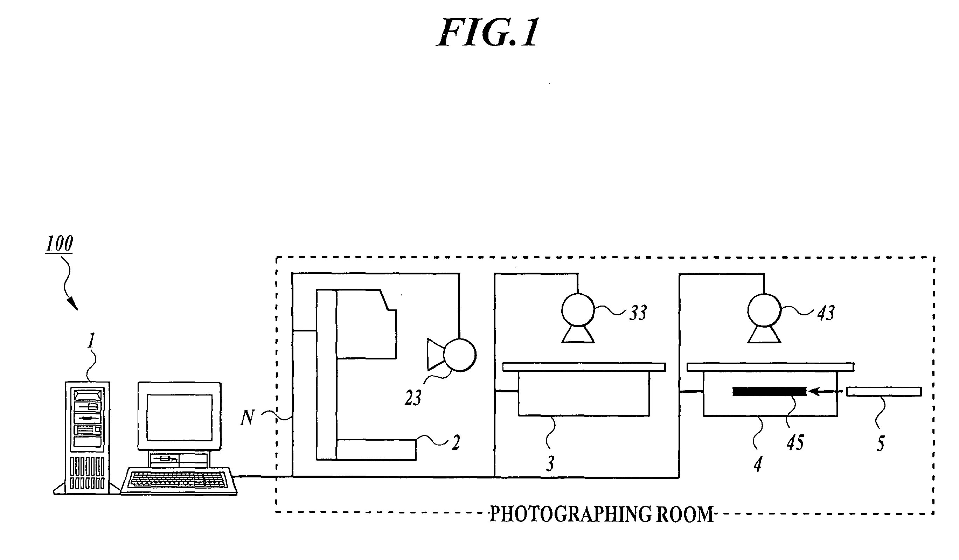

FIG. 1 is a view showing a whole structure of the medical image control system 100 of the present embodiment. As shown in FIG. 1, in the medical image control system 100, the controller 1 for functioning as a control apparatus, a medical image photographing apparatus 2 of upright-type for photographing an upright patient, a medical image photographing apparatus 3 of bucky-table-type for photographing a patient on a bucky table and a medical image photographing apparatus 4 of cassette-type using a cassette 5 incorporating a photostimulable phosphor sheet therein (hereafter, a medical image photographing apparatus is written as "reader" as abbreviation) are connected through a network N. Here, in the present embodiment, although a structure wherein one controller 1, one reader 2, one reader 3 and one reader 4 are connected through the network N is shown as an example, but the system is not limited to the mentioned structure and the number of each apparatus can be any as well as a location for placing each apparatus can be anywhere.

The network N, which is a network built with a phone line, an IDSN line, a private line, a mobile communication network, a communication satellite network, a CATV network or the like, can take various network forms such as a LAN (Local Area Network), a WAN (Wide Area Network), Internet or the like. Here, in the present embodiment, it is explained that each apparatus composing the medical image control system 100 is connected through the network N, but the connection is not limited to it. Each apparatus may be connected through a cable C of SCSI standard or the like.

The controller 1, which is connected to the readers 2, 3 and 4 placed in another room (photographing room) through the network N, controls the readers 2, 3 and 4 for photographing a medical image according to an order from an operator. Further, the controller 1 receives medical image data photographed by the readers 2, 3 and 4 through the network N or the cable C (not shown in FIGs.) and executes an image process, a display process or the like.

The reader 2, comprising a radiation source 23 (refer to FIG. 1), is the medical image photographing apparatus for photographing for an upright position. The controller 2 irradiates an X-ray from the radiation source 23 according to an order inputted from the controller 1 connected thereto through the network N. Further, a part of the irradiated radiation energy is accumulated in a photostimulable phosphor sheet as follows. Then, excitation light such as a laser beam or the like is irradiated to the photostimulable phosphor sheet to emit stimulated light sequentially. Then, the stimulated light is read by a photoelectric conversion section to obtain an image signal. Then, the reader 2 A/D converts the obtained image signal and sends the controller 1 as medical image data. Further, the reader 2 irradiates erasure light to the photostimulable phosphor sheet out of which the image signal already has been read for preparing next photographing.

The reader 3, comprising an X-ray irradiation unit 33 (refer to FIG. 1), is the medical image photographing apparatus for photographing for a bucky table position. Following the same procedure as the reader 2, the reader 3 photographs a medical image according to an order inputted from the controller 1 connected thereto through the network N. Then, the reader 3 A/D converts the obtained image signal and sends the controller 1 as medical image data.

The reader 4, comprising the radiation source 43 (refer to FIG. 1), is the medical image photographing apparatus with a cassette. Different from the readers 2 and 3, the reader 4 is an apparatus of cassette-type using a cassette 5 detachable from a main body of the apparatus. The cassette 5 incorporates the photostimulable phosphor sheet therein for accumulating a part of radiation energy. When the X-ray is irradiated from the radiation source 43 and passed through a subject placed between the radiation source 43 and the cassette 5, the photostimulable phosphor sheet accumulates a part of the irradiated radiation energy. Then, the reader 4 irradiates excitation light to the photostimulable phosphor sheet, photoelectrically converts the stimulated light emitted from the photostimulable phosphor sheet into an image signal, A/D converts the obtained image signal and sends the controller 1 as medical image data.

Next, each part composing the medical image control system 100 will be explained in detail with reference to figures.

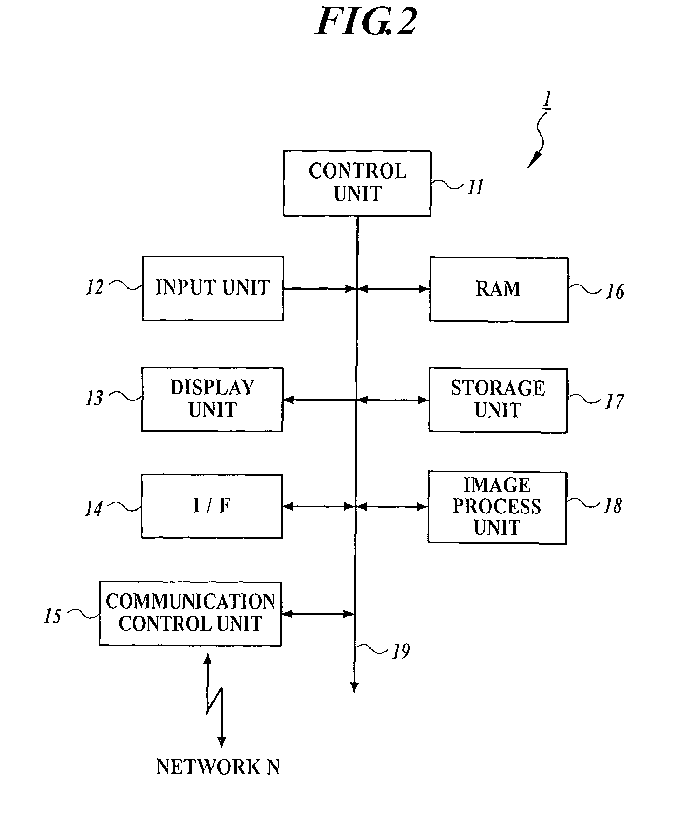

FIG. 2 is a block diagram showing a functional structure of the controller 1. As shown in FIG. 2, the controller 1 comprises a control unit 11, an input unit 12, the display unit 13, an I/F 14, a communication control unit 15, a RAM 16, a storage unit 17 and an image process unit 18. Each section is connected through a bus 19.

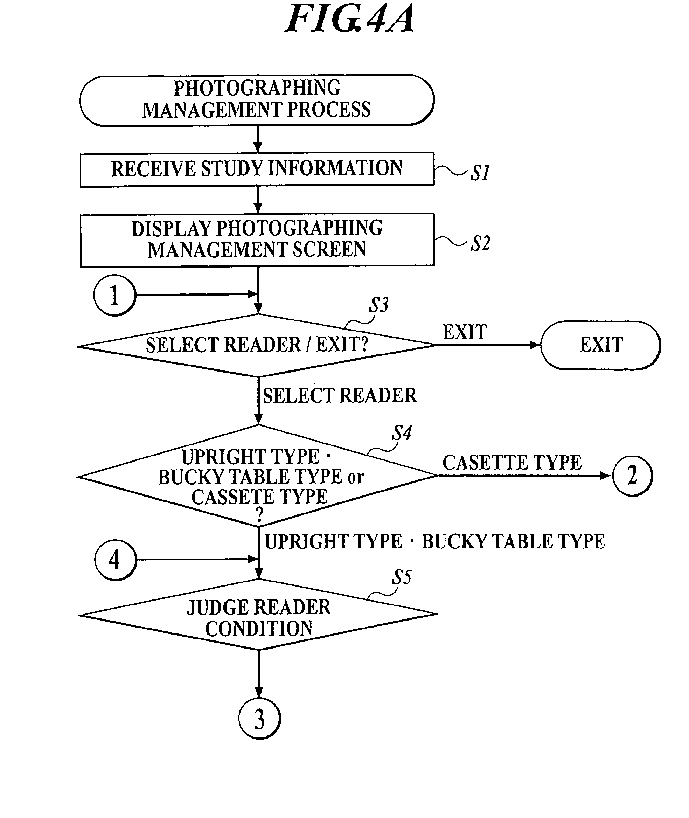

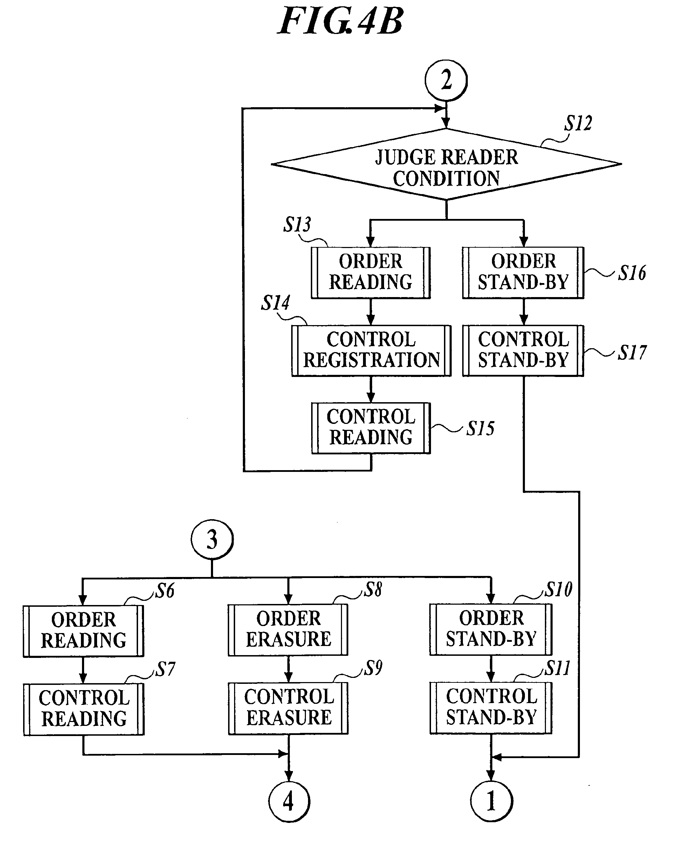

The control unit 11 is composed of a CPU (Central Processing Unit) or the like. The control unit 11 loads a system program and various control programs from the storage unit 17 and develops the programs in the RAM 16 for controlling operation to each section integrally according to the control program. Further, the control unit 11 executes various processes based on the program developed in the RAM 16. Then, a result of the process is temporarily stored in the RAM 16 and displayed on the display unit 13. Concretely, according to a photographing management process program stored in the storage unit 17 the controller 1 executes a photographing management process (refer to FIGs. 4A and 4B) as follows.

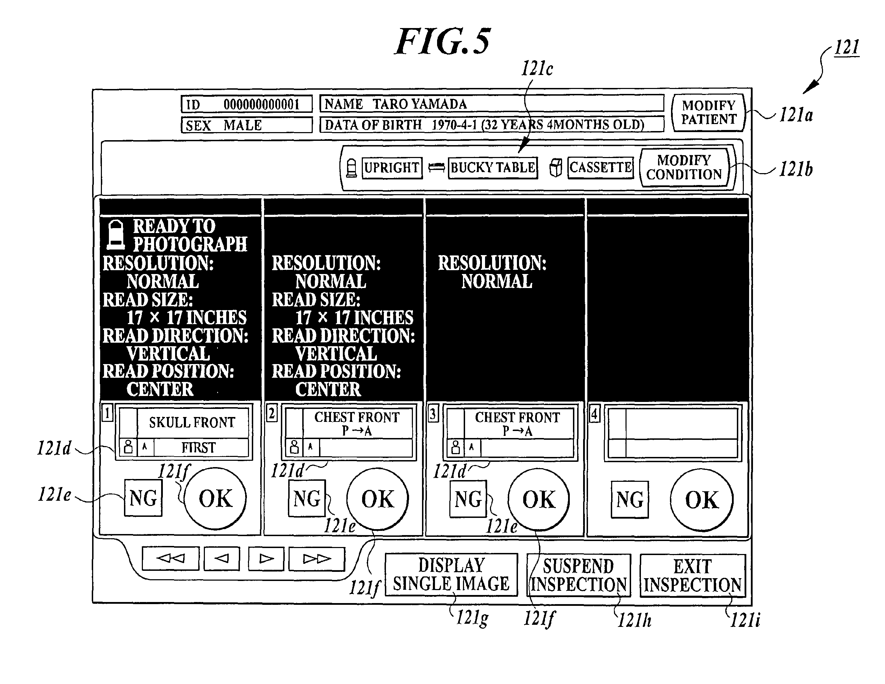

Concretely, when the control unit 11 receives study information (photographing reservation information) sent from an information system (not shown in FIGs) such as an HIS (Hospital Information System), an RIS (Radiology Information System) or the like managing information in a hospital or a department of radiology through the network N, the control unit 11 displays the photographing management screen 121 (refer to FIG. 5) capable of managing photographing operation of each of the plurality of readers 2, 3 and 4 within the same screen based on examination conditions (such as patient information, a body part examined, a reader-type to be used (the upright-type, the bucky-table-type or the cassette-type) or the like) set in the study information.

Then, when a reader is selected among the readers 2, 3 and 4 for photographing a medical image on the photographing management screen 121, the control unit 11 judges whether the selected reader is the upright-type, the bucky-table-type or the cassette-type. When the upright-type or the bucky-table-type is selected, the control unit 11 sends a command for confirming an operation state of the reader 2 or the reader 3 to the reader 2 or the reader 3 via the communication control unit 15 through the network N, and judges if the reader 2 or the reader 3 is capable of reading operation, requires erasure or is capable of shifting to be ready according to a response from the reader 2 or the reader 3. Further, when the cassette-type is selected, the control unit 11 sends the reader 4 a command for confirming an operation state of the reader 4 via the communication control unit 15 through the network N, the control unit 11 judges if the reader 4 is capable of reading operation or capable of shifting to be ready according to a response from the reader 4.

Here, being capable of reading operation means, being in a ready state and having no radiation energy remaining in a photographing panel from the last time photographing. In other words, being in a state where an erasure process has been executed. Further, requiring erasure means, being in the ready state and having radiation energy remaining in the photographing panel from the last time photographing. Further, being capable of shifting to be ready means, being in a stand-by state where power supply to a predetermined device has been stopped.

Then, when the control unit 11 judges the reader 2 or the reader 3 is capable of reading operation, according to the examination conditions set in the study information received in advance, the control unit 11 executes a control process for controlling image reading operation to the reader 2 or the reader 3. When the control unit 11 judges the reader 2 or the reader 3 requires erasure, the control unit 11 executes a control process for controlling image erasure operation to the reader 2 or the reader 3. When the control unit 11 judges the reader 2 or the reader 3 is capable of shifting to be ready, the control unit 11 executes a control process for shifting the reader 2 or the reader 3 from the stand-by state to the ready state.

Further, when the control unit 11 judges the reader 4 is capable of reading operation, the control unit executes a control process for controlling registration operation to register a barcode attached to the cassette 5 and the received study information correspondingly. Then, according to the examination conditions set in the study information, the control unit 11 executes a control process for controlling image reading operation to the reader 4. Further, when the control unit 11 judges the reader 4 is capable of shifting to be ready, the control unit 11 executes a control process for shifting the reader 4 to the ready state.

Then, when the control unit 11 finishes a set of photographing operation and a stand-by order is inputted through the input unit 12, the control unit 11 executes a control process for shifting a reader specified among the readers 2, 3 and 4 by the order, from the ready state to the stand-by state.

The input unit 12 includes a keyboard comprising a cursor key, numeric number input keys, various types of function keys or the like. The input unit 12 sends a push signal corresponding to a key pushed on the keyboard to the control unit 11. Here, the input unit 12 may comprise a pointing device such as a mouse, a touch panel or the like, or another input apparatus.

The display unit 13 is composed of an LCD (Liquid Crystal Display), a CRT (Cathode Ray Tube) or the like. According to an order of a display signal inputted from the control unit 11, the display unit 13 displays the order inputted through the input unit 12, data or the like thereon.

The I/F 14 is an interface via which the controller 1 is connected to the readers 3 and 4 through a cable C (not shown in FIGs). The cable C is a cable for sending and receiving image data having large data volume mainly. When the I/F 14 obtains medical image data from the readers 2, 3 and 4, the I/F 14 outputs the medical image data to the display unit 13, the storage unit 17, the image process unit 18 or the like according to order data inputted from the control unit 11.

The communication control unit 15 is composed of a LAN adapter, a router, a TA (Terminal Adapter) or the like. The communication control unit 15 controls communication with each apparatus connected to the network N via a communication line such as a private line, an ISDN line or the like.

The RAM (Random Access Memory) 16 forms a storage area for temporarily storing the system program and the control program which are loaded from the storage unit 17 and executable by the controller 1, input or output data, the parameter or the like, for various kinds of processes controlled to be executed by the control unit 11.

The storage unit 17 is composed of an HDD (Hard Disc Drive), a nonvolatile semiconductor memory or the like. The storage unit 17 stores the system program executed by the control unit 11, various kinds of process programs corresponding to the system program, a result of the process or the like. Further, the storage unit 17 comprises a memory medium (not shown in FIGs.) wherein a program and data are stored in advance. The memory medium is a nonvolatile memory composed of a magnetic medium, an optical medium or a semiconductor memory. The memory medium is equipped with the storage unit 17 either fixedly or detachably. The various kinds of programs are stored in a form of readable program codes. The control unit 11 sequentially executes operation according to the program codes.

The image process unit 18 applies various types of image processes such as a frequency enhancement, dynamic-range compression, gradation conversion or the like on medical image data according to the order inputted from the control unit 11. Further, the image process unit 18 executes a compression process for compressing image data with a predetermined encoding method, and an extraction process for decoding and extracting compressed image data.

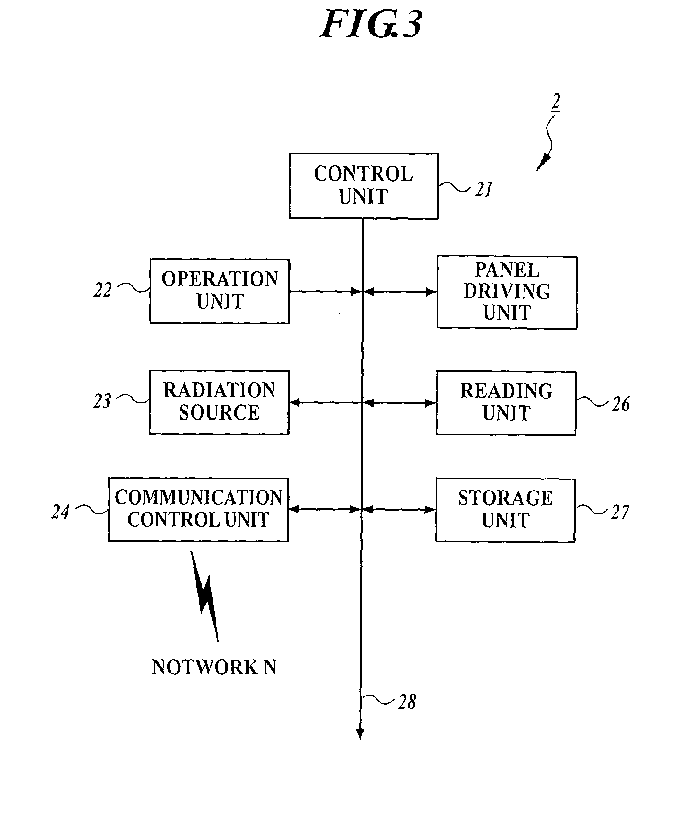

Next, a functional structure of the reader 2 of upright-type shown in FIG. 1 will be explained in detail with reference to FIG. 3. Here, regarding the reader 3, its functional structure is identical to that of the reader 2 except for its appearance being in a bed shape. Therefore, by adding a corresponding symbol to each component, the illustration and explanation of the reader 3 in detail are omitted.

As shown in FIG. 3, the reader 2 comprises a control unit 21, an operation unit 22, the radiation source 23, a communication control unit 24, a panel driving unit 25, a reading unit 26, a storage unit 27 or the like. Further, though not shown in FIGs, the reader 3 comprises a control unit 31, an operation unit 32, a radiation source 33, a communication control unit 34, a photographing panel 35, a reading unit 36, a storage unit 37 or the like.

The control unit 21 loads and executes the various system programs stored in the storage unit 27 according to an order inputted from the operation unit 22 or the controller 1 to drive each part of the reader 2 under control. Concretely, the control unit 21 executes a reading process, an erasure process and a stand-by process as follows.

At the time of execution of the reading process, when a reading order is inputted from the controller 1, the control unit 21 controls the panel driving unit 25 for moving a photographing panel (not shown in FIGs) to a position for reading based on the examination conditions such as a reading size, a reading direction, a reading part or the like. Further, the control unit 21 controls the radiation source 23 to irradiate radiation. Further, the control unit 21 controls the reading unit 26 to irradiate excitation light to the photostimulable phosphor sheet to which the radiation has been irradiated, photoelectrically converts the stimulated light emitted from the sheet and A/D converts an obtained image signal for obtaining medical image data.

Further, at the time of execution of the erasure process, when an erasure order is inputted from the controller 1, the control unit 21 controls the reading unit 26 to irradiate erasure light to the photostimulable phosphor sheet out of which the image signal already has been read, for discharging radiation energy remaining in the plate.

Further, at the time of execution of the stand-by process, when a stand-by order is inputted from the controller 1, the control unit 21 examines a present state of the apparatus. When the apparatus is presently in the stand-by state, the control unit 21 shifts the present state to the ready state where a process can be executed as soon as an order is inputted from the controller 1 or the operation unit 22. On the other hand, when the apparatus is presently in the ready state, the control unit 21 stops the power supply to a predetermined device and shifts the present state to the stand-by state in order to reduce power consumption of the predetermined device.

The operation unit 22 is composed of an operation panel comprising numeric number input keys, cursor keys, various function keys or the like. The operation unit 22 outputs a push signal according to a key pushed on the operation panel to the control unit 21. Further, the push signal is outputted to the controller 1 via the communication control unit 24 as an operation signal.

The radiation source 23 conically irradiates radiation to a subject according to control of the control unit 21 based on radiation dose and irradiation timing ordered by the controller 1.

The communication control unit 24 is composed of a LAN adapter, a modem, a TA (Terminal Adapter) or the like. The communication control unit 24 controls communication with the controller 1 connected to the network N composed of a private line, an ISDN line or the like. The communication control unit 24 outputs a control signal inputted from the controller 1 to the control unit 21, and sends image data inputted from the reading unit 26 and the operation signal inputted through the operation unit 22 to the controller 1.

The panel driving unit 25 drives the photographing panel (not shown in FIGs) to a predetermined position for reading according to the control signal inputted from the control unit 21. The photographing panel has a structure wherein the photostimulable phosphor sheet is incorporated in a photographing platform (not shown in FIGs) in advance. The photographing panel accumulates X-ray radiation energy passed through the subject such as a human body or the like and its surroundings out of the X-ray irradiated from the radiation source 23 for recording an X-ray image of the subject. When the excitation light such as visible light or the like is irradiated to the photostimulable phosphor sheet, the accumulated energy is liberated and the stimulated light is emitted. By taking advantage of the phenomenon, the reading unit 26 performs reading an image recorded in the photostimulable phosphor sheet and erasure on the photostimulable phosphor sheet.

The reading unit 26 is composed of an excitation light generator, a photoelectric conversion device, an A/D converter or the like. The reading unit 26 irradiates the excitation light to the photographing panel in which a photostimulable phosphor layer is formed, for scanning according to the control signal inputted from the control unit 21, and photoelectrically converts the stimulated light emitted from the photographing panel for generating an image signal. Further, The reading unit 26 A/D converts the generated image signal and outputs the converted image data to the communication control unit 24 as original image data. Further, according to an order inputted from the controller 1 via the communication control unit 24, the reading unit 26 irradiates erasure light for erasing image data remaining in the photostimulable phosphor plate. Here, reading the image signal is not limited to the above-mentioned method using the photostimulable phosphor layer, and a method using an FPD (Flat Panel Detector) with a semiconductor detector may be used.

Next, regarding the reader 4, since its structure is approximately identical to that of the readers 2 and 3 as mentioned above, by adding corresponding symbols to identical components, the illustration and explanation of the reader 4 in detail are omitted. Concretely, the reader 4 is composed of a control unit 41, an operation unit 42, the radiation source 43, a communication control unit 44, a reading unit 46, a storage unit 47 or the like. Further, the reader 4 does not comprise a photographing panel but a cassette holding unit 45 for connecting a detachable cassette.

The control unit 41 of the reader 4 executes the reading process and the stand-by process according to the order from the controller 1 as with the above-mentioned case of the control unit 21 of the reader 2. Further, as a process peculiar to the reader 4, which is the medical image photographing apparatus of cassette-type, when the reading order is inputted, prior to execution of the reading process, the control unit 41 executes a registration process to read a barcode (an ID number of the cassette) attached to the cassette 5 which is to be used for photographing by a reading apparatus (not shown in FIGs), and store the barcode and the study information correspondingly in the RAM (not shown in FIGs).

The cassette holding unit 45 is a holding section for setting the cassette 5 in the reader 4. The cassette 5, wherein the photostimulable phosphor plate is contained in a portable and flat covering, is set in the photographing platform detachably. The cassette 5 accumulates X-ray radiation energy passed through the subject such as a human body or the like and its surroundings out of the X-ray irradiated from the radiation source 43 for recording an X-ray image of the subject.

In other words, the reader 4 equips the portable cassette 5 incorporating the photostimulable phosphor plate therein in the cassette holding unit 45 for photographing. Then, after the photographing, the cassette 5 with which the photographing has been performed is replaced with a new cassette 5. As a result, it is possible to photograph continuously without the erasure process required.

Next, operation of the present embodiment will be explained.

As a premise of explanation of the operation, programs for executing processes as follows are stored as program codes which are readable by a computer in the storage unit 17. Further, the control unit 11 sequentially executes operation according to the program codes.

A photographing management process executed by the controller 1 shown in FIG. 2 will be explained with reference to a flow chart shown in FIGs. 4A and 4B. Here, regarding the process, a case that the readers 2, 3 and 4 respectively perform the medical image photographing operation will be explained.

In FIG. 4A, when the control unit 11 receives the study information via the communication control unit 15 through the network N from an external information system (not shown in FIGs) managing information in a hospital or a department of radiology (Step S1) the control unit 11 displays the photographing management screen 121 as shown in FIG. 5 capable of managing photographing operation to each of the plurality of readers 2, 3 and 4 within the same screen based on the examination conditions (patient information, a body part examined, a reader-type to be used (the upright-type, the bucky-table-type or the cassette-type) or the like) set in the study information on the display unit 13 (Step S2).

On the photographing management screen 121 shown in FIG. 5, the control unit 11 displays the patient information such as a patient ID, a patient name, a sex and a date of birth based on the inputted study information at the top of the screen, and also displays a modify patient button 121a for shifting to a modify screen for modifying the patient information at the right of the patient information. Further, below the patient information, the control unit 11 displays a modify condition button 121b for shifting to a modify screen for modifying the examination conditions included in the study information, and a reader selecting button 121c for selecting a reader among the upright-type, the bucky-table-type and the cassette-type for reading a medical image.

Further, in the middle of the screen, photographing operation management areas are provided, the photographing operation management areas wherein a list of the examination conditions or the like along with an order number are displayed from left to right in sequence of registration of the study information of a patient. On an order icon 121d placed in the photographing operation management area, the examination conditions, outputting conditions or the like with simple symbols and figures are displayed so that the photographic technician can easily comprehend registered contents of the study information. For example, a display of "SKULL FRONT" indicates examination conditions of a body part examined and a photographing direction, and a display of "A" indicates output conditions of rotation and reversal. Further, when photographing is completed and medical image data is received from the readers 2, 3 and 4, a part of the photographing operation management area (for example, a black part of FIG. 5) becomes a medical image display area for displaying the medical image of each of the readers 2, 3 and 4 corresponding to the study information.

Here, the leftmost area in FIG. 5 is the photographing operation management area and the medical image display area for the reader 2, the second leftmost area is the photographing operation management area and the medical image display area for the reader 3, and the third leftmost area is the photographing operation management area and the medical image display area for the reader 4. The rightmost area is displayed as unavailable since its medical image display area is not used.

Further, in FIG. 5, below each order icon 121d, an "NG" button 121e and an "OK" button 121f are displayed as order buttons for ordering medical image reading operation to each of the readers 2, 3 and 4. The "NG" button 121e is operated when the medical image reading operation to the readers 2, 3 and 4 is abnormal and therefore it is necessary to abort the operation. The "OK" button 121f is operated when the medical image reading operation to the readers 2, 3 and 4 is normal and therefore it is necessary to exit.

Further, in FIG. 5, a "DISPLAY SINGLE IMAGE" button 121g is operated for displaying any one of images read by the readers 2, 3 and 4. A "SUSPEND INSPECTION" button 121h is operated for suspending the medical image reading operation to each of the readers 2, 3 and 4 according to the study information in question. An "EXIT INSPECTION" button 121i is operated for exiting the medical image reading operation to each of the readers 2, 3 and 4 according to the study information in question all at once.

Next, in FIGs. 4A and 4B, the control unit 11 judges whether the reader selecting button 121c or the "EXIT INSPECTION" button 121i is operated (Step S3). When a reader is selected among the readers 2, 3 and 4 for photographing, the control unit 11 judges if the selected reader is the upright-type, the bucky-table-type or the cassette-type (Step S4).

When the upright-type or the bucky-table-type is selected, the control unit 11 sends a command for confirming an operation state of the reader 2 or the reader 3 to the reader 2 or the reader 3 via the communication control unit 15 through the network N. Then, the control unit 11 judges if the reader 2 or the reader 3 is capable of the reading operation, requires the erasure or is capable of shifting to be ready according to the response from the reader 2 or the reader 3 (Step S5).

Then, when the control unit 11 judges the reader 2 or the reader 3 is capable of the reading operation, the control unit 11 executes a control process for controlling the image reading operation to the reader 2 or the reader 3 according to the examination conditions set in the study information received in advance (Steps S6, S7). When the control unit 11 judges the reader 2 or the reader 3 requires erasure, the control unit 11 executes a control process for controlling the image erasure operation to the reader 2 or the reader 3 (Steps S8, S9). When the control unit 11 judges the reader 2 or the reader 3 is capable of shifting to be ready, the control unit 11 executes a control process for shifting the reader 2 or the reader 3 to the ready state (Steps S10, S11).

Further, when the cassette-type is selected in Step S4, the control unit 11 sends a command for confirming an operation state of the reader 4 to the reader 4 via the communication control unit 15 through the network N. Then, the control unit 11 judges if the reader 4 is capable of the reading operation or is capable of shifting to be ready according to the response from the reader 4 (Step S12).

Then, when the control unit 11 judges the reader 4 is capable of the reading operation, the control unit 11 performs control of registering the barcode attached to the cassette 5 set in the reader 4 and the received study information correspondingly, and then the control unit 11 executes a control process for controlling the image reading operation to the reader 4 according to the examination conditions set in the study information (Steps S13, S14, S15). Further, when the control unit 11 judges the reader 4 is capable of shifting to be ready, the control unit 11 executes a control process for shifting the reader 4 to the ready state (Steps S16, S17).

Here, when the above-mentioned reading operation to the reader 2 or the reader 3 is completed, the control unit 11 receives medical image data sent from the reader 2 or the reader 3 via the communication control unit 15 through the network N. Then, after the image process unit 18 applies a predetermined image process on the received medical image data, the control unit 11 displays the image-processed data on the medical image display area located leftmost or second leftmost in the photographing management screen 121 in FIG. 5.

Then, according to a determination made by an operator (the photographic technician) with reference to the displayed medical image whether or not the image reading operation in question is normal, either the "NG" button 121e or the "OK" button 121f is operated for ordering the medical image reading operation to the reader 2 or the reader 3. When the "NG" button 121e is operated, the control unit 11 executes the reading operation control process to the reader 2 or the reader 3 once again. When the "OK" button 121f is operated, the control unit 11 executes the image erasure operation control process to the reader 2 or the reader 3.

Further, in the case of the reader 4 of cassette-type, after the cassette 5 is set in a cassette reader (not shown in FIGs) for reading, when the control unit 11 receives the medical image data sent from the cassette reader via the communication control unit 15 through the network N, the image process unit 18 applies a predetermined image process on the received medical image data, and then the image-processed image data is displayed on the medical image display area located third leftmost in the photographing management screen 121 shown in FIG. 5.

Then, according to a determination made by the operator (the photographic technician) with reference to the displayed medical image whether or not the image reading operation in question is normal, either the "NG" button 121e or the "OK" button 121f is operated for ordering the medical image reading operation to the reader 4. When the "NG" button 121e is operated, the control unit 11 executes the reading operation control process to the reader 4 once again. When the "OK" button 121f is operated, the control unit 11 executes the stand-by operation control process to the reader 4 for shifting the reader 4 to the stand-by state.

In FIGs. 4A and 4B, after either the reading operation control process or the erasure operation control process to the reader 2 or the reader 3 is completed, the control unit 11 goes back to Step S5 for judging the operation state of the reader 2 or the reader 3. When the study information has more orders for photographing medical images, the control unit 11 executes the reading operation control process and the erasure operation control process over again. In other words, the control unit 11 repeatedly executes the processes from Step S5 to Step S9 until completion of photographing all medical images set in the study information to the reader 2 or the reader 3.

Then, when the medical image photographing process to the reader 2 or the reader 3 is completed, the control unit 11 executes the stand-by operation control process for shifting the reader 2 or the reader 3 to the stand-by state (Step S10, S11), and goes back to Step S3 for judging whether to select a reader or exit inspection.

Further, when execution of the reading operation control process to the reader 4 is completed, the control unit 11 goes back to Step S12 for judging the operation state of the reader 4. When the study information has more orders for photographing medical images, the control unit 11 executes the reading operation control process over again. In other words, the control unit 11 repeatedly executes the processes from Step S12 to Step S15 until completion of photographing all medical images set in the study information to the reader 4.

Then, when the medical image photographing process to the reader 4 is completed, the control unit 11 executes a stand-by operation control process for shifting the reader 4 to the stand-by state (Step S16, S17), and goes back to Step S3 for judging whether to select a reader or exit inspection.

As mentioned above, the controller 1, which is connected to the plurality of readers 2, 3 and 4 having different control patterns to one another, displays the photographing management screen 121 capable of managing the plurality of readers 2, 3 and 4 simultaneously on the display unit 13, and executes the reading operation control process, the erasure operation control process or the stand-by operation control process according to various orders inputted through the photographing management screen 121 and the ordered reader for controlling the photographing operation to the readers 2, 3 and 4.

Therefore, in the medical image control system 100, in a case that the plurality of readers 2, 3 and 4 are provided, it is not necessary to provide a controller for each of the readers 2, 3 and 4, and therefore one controller 1 can control the plurality of readers 2, 3 and 4 simultaneously. Accordingly, when the operator (the photographic technician) photographs medical images by using the plurality of readers 2, 3 and 4 to one patient, it is possible to provide him a simple control operation of each of the readers 2, 3 and 4 and shorten a time period for photographing. For example, when inputted information is incorrect, it is possible to correct the information to the plurality of readers 2, 3 and 4 simultaneously by correcting the information in one controller 1. As a result, it is possible to save labor and time in the correcting operation.

Further, since it is possible to display the study information to the plurality of readers 2, 3 and 4 within one screen simultaneously, it is possible to provide a user-friendly, convenient controller 1 without switching a screen for each of the readers 2, 3 and 4. Further, since it is possible to photograph a medical image while a next operation plan is confirmed, it is possible to photograph efficiently as well as reduce strain on a patient. Further, since the photographing operation management areas for each of the readers 2, 3 and 4 are individually displayed, the operator can easily recognize the study information to each of the readers 2, 3 and 4. As a result, it is possible to provide a display screen having good visibility.

Alternatively, since one controller 1 can control the plurality of readers 2, 3 and 4, it is possible to save maintenance cost for the plurality of readers 2, 3 and 4, as well as space for installing the plurality of readers 2, 3 and 4.

Here, description of the present embodiment as described above is an example of a medical image control apparatus and a medical image control system appropriate in regard to the present invention, and the apparatus or the system is not limited to it.

For example, although it has been explained that the medical image control system 100 comprises a medical image photographing apparatus including a medical image apparatus of upright-type, bucky-table-type and cassette-type, the system is not limited to it and therefore it is possible to comprise various reader-types of medical image photographing apparatuses. Alternatively, the system may have a structure wherein the controller 1 can manage medical image photographing apparatuses made by different manufacturers, within the same screen.

And otherwise, each component, a detailed structure of component parts of the controller 1, the readers 2, 3 and 4, and detailed operation of the medical image control system 100 of the present embodiment may be changed appropriately as long as the change does not depart from essence of the present invention.

The entire disclosure of Japanese Patent Application No. Tokugan 2002-237690 filed on August 16, 2002 including specification, claims, drawings and summary are incorporated herein by reference in its entirety.

## Claims

1. A medical image control apparatus for controlling a cassette-type medical image photographing apparatus and at least one of an upright-type medical image photographing apparatus and a bucky-table-type medical image photographing apparatus, the medical image control apparatus comprising:
a display control section for displaying a photographing management screen on a display section, the photographing management screen capable of managing medical image photographing operation of each of the cassette-type medical image photographing apparatus and the at least one of an upright-type medical image photographing apparatus and a bucky-table-type medical image photographing apparatus based on photographing reservation information therewithin, and
a photographing operation control section for controlling medical image photographing operation of a medical image photographing apparatus ordered on the photographing management screen,
wherein the display control section individually displays medical images within photographing operation management areas corresponding to the cassette-type medical image photographing apparatus and the at least one of an upright-type medical image photographing apparatus and a bucky-table-type medical image photographing apparatus, the medical images read by the cassette-type medical image photographing apparatus and the at least one of an upright-type medical image photographing apparatus and a bucky-table-type medical image photographing apparatus corresponding, and
the photographing operation control section controls medical image photographing operation corresponding to each of the cassette-type medical image photographing apparatus and the at least one of an upright-type medical image photographing apparatus and a bucky-table-type medical image photographing apparatus, according to an order on each of the photographing operation management areas within the photographing management screen.

2. The medical image control apparatus of claim 1, wherein the order on each of the photographing operation management areas includes at least one of an order of exiting operation normally and an order of aborting operation.

3. The medical image control apparatus of claim 1, further comprising a communication control section for receiving the photographing reservation information from an information system.

4. The medical image control apparatus of claim 1 further comprising a display section for displaying any one of the medical images displayed on the photographing management screen by operation.

5. The medical image control apparatus of claim 3,
wherein the photographing operation control section judges whether the cassette-type medical image photographing apparatus capable of performing reading operation when the cassette-type medical image photographing apparatus is selected by operation, and
the photographing operation control section controls registration of a code of a cassette set in the cassette-type medical image photographing apparatus and the photographing reservation information received, the code and the photographing reservation information are related, when judging the cassette-type medical image photographing apparatus is capable of performing the reading operation.

6. The medical image control apparatus of claim 1, wherein the photographing operation control section controls reading operation of the cassette-type medical image photographing apparatus and the at least one of an upright-type medical image photographing apparatus and a bucky-table-type medical image photographing apparatus according to the photographing reservation information.

7. A medical image control system comprising a cassette-type medical image photographing apparatus and at least one of an upright-type medical image photographing apparatus and a bucky-table-type medical image photographing apparatus and a medical image control apparatus for controlling the cassette-type medical image photographing apparatus and the at least one of an upright-type medical image photographing apparatus and a bucky-table-type medical image photographing apparatus, the medical image control apparatus comprising:
a display control section for displaying a photographing management screen on a display section, the photographing management screen capable of managing medical image photographing operation of each of the cassette-type medical image photographing apparatus and the at least one of an upright-type medical image photographing apparatus and a bucky-table-type medical image photographing apparatus based on photographing reservation information therewithin, and
a photographing operation control section for controlling medical image photographing operation of a medical image photographing apparatus ordered on the photographing management screen,
wherein the display control section individually displays medical images within photographing operation management areas corresponding to the cassette-type medical image photographing apparatus and the at least one of an upright-type medical image photographing apparatus and a bucky-table-type medical image photographing apparatus, the medical images read by the cassette-type medical image photographing apparatus and the at least one of an upright-type medical image photographing apparatus and a bucky-table-type medical image photographing apparatus corresponding, and
the photographing operation control section controls medical image photographing operation corresponding to each of the cassette-type medical image photographing apparatus and the at least one of an upright-type medical image photographing apparatuses and a bucky-table-type medical image photographing apparatus, according to an order on each of the photographing operation management areas within the photographing management screen.

8. The medical image control system of claim 7, wherein the order on each of the photographing operation management areas includes at least one of an order of exiting operation normally and an order of aborting operation.

9. The medical image control system of claim 7, wherein the medical image control apparatus further comprises a communication control section for receiving the photographing reservation information from an information system.

10. The medical image control system of claim 7, wherein the medical image control apparatus further comprises a display section for displaying any one of the medical images displayed on the photographing management screen by operation.

11. The medical image control system of claim 9, wherein the photographing operation control section judges whether the cassette-type medical image photographing apparatus is capable of performing reading operation when the cassette-type medical image photographing apparatus is selected by operation, and
the photographing operation control section controls registration of a code of a cassette set in the cassette-type medical image photographing apparatus and the photographing reservation information received, the code and the photographing reservation information are related, when judging the cassette-type medical image photographing apparatus is capable of performing the reading operation.

12. The medical image control system of claim 7, wherein the photographing operation control section controls reading operation of the cassette-type medical image photographing apparatus and the at least one of an upright-type medical image photographing apparatus and a bucky-table-type medical image photographing apparatus according to the photographing reservation information.

13. A program, when the program is loaded onto a medical image control apparatus for controlling a cassette-type medical image photographing apparatus and at least one of an upright-type medical image photographing apparatus and a bucky-table-type medical image photographing apparatus, the program making the medical image control apparatus execute:
displaying a photographing management screen on a display section, the photographing management screen capable of managing medical image photographing operation of each of the cassette-type medical image photographing apparatus and the at least one of an upright-type medical image photographing apparatus and a bucky-table-type medical image photographing apparatus based on photographing reservation information therewithin, and
controlling medical image photographing operation of a medical image photographing apparatus ordered on the photographing management screen,
wherein the displaying a photographing management screen on a display section includes individually displaying medical images within photographing operation management areas corresponding to the cassette-type medical image photographing apparatus and the at least one of an upright-type medical image photographing apparatus and a bucky-table-type medical image photographing apparatus, the medical images read by the cassette-type medical image photographing apparatus and the at least one of an upright-type medical image photographing apparatus and a bucky-table-type medical image photographing apparatus corresponding, and
the controlling medical image photographing operation of a medical image photographing apparatus includes controlling medical image photographing operation corresponding to each of the cassette-type medical image photographing apparatus and the at least one of an upright-type medical image photographing apparatuses and a bucky-table-type medical image photographing apparatus, according to an order on each of the photographing operation management areas within the photographing management screen.

14. The program of claim 13, wherein the order on each of the photographing operation management areas includes at least one of an order of exiting operation normally and an order of aborting operation.

15. The program of claim 13, further making the medical image control apparatus execute receiving the photographing reservation information from an information system.

16. The program of claim 13, further making the medical image control apparatus execute displaying any one of the medical images displayed on the photographing management screen by operation.

17. The program of claim 15,
wherein the controlling medical image photographing operation of a medical image photographing apparatus includes judging whether the cassette-type medical image photographing apparatus is capable of performing reading operation when the cassette-type medical image photographing apparatus is selected by operation, and
the controlling medical image photographing operation of a medical image photographing apparatus includes controlling registration of a code of a cassette set in the cassette-type medical image photographing apparatus and the photographing reservation information received, the code and the photographing reservation information are related, when judging the cassette-type medical image photographing apparatus is capable of performing the reading operation.

18. The program of claim 13, wherein the controlling medical image photographing operation of a medical image photographing apparatus includes controlling reading operation of the cassette-type medical image photographing apparatus and the at least one of an upright-type medical image photographing apparatus and a bucky-table-type medical image photographing apparatus according to the photographing reservation information.
